# EUROPÄISCHE PATENTSCHRIFT

(11) **EP 1 235 179 B1**
(45) Veröffentlichungstag und Bekanntmachung des Hinweises auf die Patenterteilung: **29.08.2007**
(21) Anmeldenummer: 01124641.0
(22) Anmeldetag: 16.10.2001
(51) Int. Cl.: G06F 19/00

(54) **System zur Erfassung und Speicherung personenspezifischer Daten und entsprechendes Speicherelement sowie Verfahren zur Rettung und/oder medizinischen Versorgung von Lebewesen im Notfall**
System for collecting and storing of personspecific data and associated memoryelement and a method for saving of or medical healthcare for a living being in distress
Système de collection et de stockage de données personnelles et élément de mémoire associé et méthode de sauvetage et de traitement médical d'un être vivant en urgence

(30) Priorität: 10.01.2001 DE 10100722
(43) Veröffentlichungstag der Anmeldung: 28.08.2002
(60) Teilanmeldung: 06003021.0
(73) Patentinhaber: Rehwald, Jörg, Dr., 20249 Hamburg (DE)
(72) Erfinder: Rehwald, Jörg, Dr., 20249 Hamburg (DE)
(74) Vertreter: Kuhnen & Wacker

(56) Entgegenhaltungen:
- EP-A- 0 759 600
- WO-A-96/08755
- WO-A-97/04712
- GB-A- 2 285 135
- US-A- 5 659 741

## Beschreibung

Die Erfindung betrifft ein Speicherelement zur Speicherung personenspezifischer Daten mit wenigstens zwei Speicherbereichen und ein System zur Erfassung und Speicherung personenspezifischer Daten umfassend einen Computer, ein entsprechendes Speicherelement und eine Verbindungseinrichtung. Die Erfindung betrifft ferner ein System zur Erfassung und Speicherung personenspezifischer Daten umfassend einen ersten Computer, der verschiedene Speicherbereiche auf einem Computerspeicherelement beinhaltet. Im weiteren betrifft die Erfindung ein Verfahren zur Rettung und/oder medizinischen Versorgung von Lebewesen im Notfall, insbesondere verunfallter Menschen, sowie ein Computerprogrammprodukt zur Durchführung des Verfahrens.

Unter personenspezifischen Daten sind insbesondere Daten von Menschen gemeint, aber auch Daten von Tieren.

Speicherelemente und Systeme zur Speicherung personenspezifischer Daten sind beispielsweise im Gesundheitswesen bekannt. Es ist heutzutage üblich, Stammdaten eines Patienten auf einer Versichertenkarte zu speichern, wobei der Patient diese Versichertenkarte mit sich führt und beispielsweise bei einem Arztbesuch diese Versichertenkarte abgibt, damit die indem Speicherelement gespeicherten Daten in ein Computersystem beim Arzt eingelesen werden können. Die Stammdaten umfassen hierbei insbesondere den Namen, die Versicherung und das Alter des Patienten.

Für den Arzt gibt es schon Systeme, in denen auf einem Computer weitere Daten von den Patienten gespeichert sind, wie beispielsweise schon durchgeführte Diagnosen, Laboruntersuchungen und bisher ausgestellte Rezepte. Insbesondere die Anamnese ist für den behandelnden Arzt von sehr hoher Wichtigkeit. Nach der neueren Diagnose des Arztes stellt dieser üblicherweise ein Rezept aus, mit dem der Patient zur Apotheke geht, um dieses einzulösen.

Diese Methode bzw. dieses System ist relativ aufwendig. Der Apotheker muß nun die Daten aus dem Rezept in sein Computersystem einlesen, um eine entsprechende Abrechnung vorzunehmen. Somit ist die herkömmliche Vorgehensweise mit der Ausfüllung von papiernen Rezepten zeitaufwendig.

Bei einem Notfall, beispielsweise bei einem Unfall, ist es nur sehr schwierig für den behandelnden Arzt eine Anamnese des Notfallpatienten zu erhalten. Nur wenige Notfallpatienten besitzen einen Unfallpaß. Die Informationen aus dem Unfallpaß sind meistens auch sehr unvollständig.

Darüber hinaus bestehen Probleme bei der Rettung von verunglückten oder ohnmächtiger Menschen, da zur Rettung dieser Personen viel Zeit vergeht, bis die Personen eine richtige medizinische Erstversorgung bekommen, da in der Regel die Personen zunächst bei diesem Notfall nicht in eine entsprechende Klinik eingeliefert werden, sondern vielmehr zunächst nach Einlieferung in eine allgemeine Klinik behandelt werden. Erst danach wird über eine spezielle Behandlung entschieden. Ferner vergeht in dieser Notfallsituation auch viel Zeit, bis Daten über und zu dem Patienten überhaupt vorliegen. Dies tritt insbesondere dann ein, wenn das Unfallopfer ohnmächtig und/oder nicht ansprechbar ist.

Systeme zur Speicherung personenspezifischer Daten werden beispielsweise in der WO 96/08755 A1 bzw. der EP 0 781 428 B1 diskutiert. Dabei wird ein Personendaten-Archivierungssystem mit zumindest zwei transportablen, persönlichen Speichereinrichtungen offenbart. Die Speichereinrichtungen sind zum Speichern und Aufbewahren von Personendaten beim Inhaber geeignet. Den Speichereinrichtungen sind ferner Berechtigungsprüfeinrichtungen zugeordnet, mittels derer nur in Abhängigkeit von einer positiven Zugangsberechtigung Zugriff auf zumindest einen Teil der gespeicherten Daten freigegeben wird.

Das Personendaten-Archivierungssystein aus der WO 96/08755 A1 sieht dabei zwei physikalische Speicherlemente zur Archivierung der Personendaten vor, wobei ein erstes Speicherelement beispielsweise eine Prozessorchipkarte seinkann, die einen Notfalldatenspeicherbereich enthält, in welchem Verwaltungsdaten und Daten eines Notfallausweises, sowie zusätzlich Übersichtsdaten gespeichert sein können, während das andere Speicherelement ein herkömmlicher, handelsüblicher Datenträger, beispielsweise eine Diskette, eine CD-ROM oder dergleichen sein kann, auf dem Detaildaten gespeichert werden können. Auf die Daten, welche auf dem ersten Speicherelement gespeichert wind, kann hierbei mittels einer Lesegeräterkennung zugegriffen werden, während auf die Daten des zweiten Speicherelements nur in Verbindung mit dem ersten Speicherelement zugegriffen werden kann. Zudem können die Daten des zweiten Speicherelements auch zusätzlich per PIN geschützt sein.

Weiterhin offenbart die WO 97/21118 A1 einen berührungslosen elektronischen Transponder mit einer integrierten Antennenschaltung in Kartenform, wobei mittels elektromagnetischer Strahlung dabei Informationen wie beispielsweise Kreditkarteninformationen, medizinische Daten oder ähnliches von der Karte gelesen werden können.

Ferner offenbart die WO 97/04712 A1 ein System zur personenbezogenen Übertragung und Speicherung medizinisch-klinischer Daten und eine IC-Karte für ein derartiges System. Die Karte weist hierbei einen als Ringspeicher ausgebildeten mikroprozessorgesteuerten Schreib-Lesespeicher auf. In diesem Speicher werden personenbezogene medizinische Daten, insbesondere Allergien, Dauermediaktionen und Akutmediaktionen verschlüsselt gespeichert, wobei die Speicher- und Zugriffsbereiche in administrative Personendaten, medizinische Gesamtdaten und medizinische Notfalldaten aufgeteilt sein können, denen jeweils entsprechende Zugriffsberechtigungsstufen wie beispielsweise eine vom Karteninhaber selbst generierte PIN zugeordnet werden können, wobei ein Arzt mittels einer Arztkarte die PIN des Karteninhabers übergehen kann, um die entsprechenden Informationen auszulesen.

Mittels eines externen Rechners können die auf der Karte gespeicherten Daten gelesen, geschrieben und ausgewertet werden, wobei hierfür neben den auf der Karte gespeicherten Daten zusätzlich im entsprechenden Praxis- bzw. Klinikverwaltungssystem gespeicherte Daten benötigt werden. Dabei kann eine Überprüfung durchgeführt werden, ob mögliche Kontraindikationen oder Arzneimittel-Interaktionen vorliegen. Zusätzlich kann per Datenaustausch über eine Schnittstelle auf die externen Datenspeicher mit Diagnose- und Befund-Thesauri zurückgegriffen werden, so daß das Behandlungsrisiko für den Patienten verringert werden kann.

Es ist demgemäß eine Aufgabe der vorliegenden Erfindung, die medizinische Versorgung im Allgemeinen und die Rettung von verunfallten Menschen oder Menschen im Notfall im Speziellen zu verbessern, wobei hierbei spezifische Daten über den Patienten zur Verfügung gestellt werden sollen, ohne hierfür für den erforderlichen Datenaustausch zwischen einer versorgenden Person und dem Patienten eine Chipkarte oder dergleichen mit entsprechend darauf hinterlegten Daten vom Patienten ausgehändigt bekommen zu müssen, wobei hinsichtlich der auf der Chipkarte oder dergleichen gespeicherten Daten des Patienten geltende datenschutzrechtliche Belange Berücksichtigung finden sollen, so dass die Daten des Patienten nur von dem für den jeweiligen Versorgungsschritt zuständigen Personenkreis eingesehen und bearbeitet werden können.

Gelöst wird diese Aufgabe durch ein Speicherelement zur Speicherung personenspezifischer Daten mit den Merkmalen nach Anspruch 1.

Durch das erfindungsgemäße Speicherelement ist es möglich, daß beispielsweise Hilfspersonal beim Arzt nur einen Speicherbereich lesen können während mit einer speziellen Zugangsberechtigung des Arztes weitere Speicherbereiche für den Arzt lesbar und beschreibbar sein können. Mit einem derartigen Speicherelement ist es möglich, so wichtige Diagnosen wie die Blutgruppe und Allergien mit einem Zugangsschutz, den beispielsweise nur Ärzte besitzen, zugänglich zu machen. Wenn das Speicherelement auch einen Speicherbereich umfaßt, auf dem ein Rezeptteil vorgesehen ist, können die Rezeptdaten von einem Apotheker ohne weiteres und auf einfache Art und Weise übernommen werden, so daß Zeit und damit Kosten gespart werden.

Es kann auch vorgesehen sein, weitere Daten im Speicher vorzusehen, die vom Patienten selbst bestimmt werden und die bspw. mit dem Arzt zusammen lesbar sind.

Vorzugsweise umfaßt das Speicherelement wenigstens einen Speicherbereich der ein ROM ist, also einen Bereich, der nur lesbar ist und wenigstens einen weiteren Speicherbereich, der ein RAM ist, also einen Bereich, der ein Lese- und Schreibbereich ist. In dem ROM sind dann beispielsweise Stammdaten wie der Name, Adresse, die Daten der Krankenversicherung, Daten zum nächsten Angehörigen oder zu einem Hausarzt und möglicherweise ein Paßfoto gespeichert. Es können auch andere personenspezifische Daten, insbesondere biometrische Daten, bspw. ein Abbild der Iris oder ein Fingerabdruck gespeichert sein. Im RAM, das in mehrere Bereiche oder Felder aufgeteilt sein kann, können beispielsweise Diagnosen gespeichert werden wie Herzerkrankungen, Stoffwechselerkrankungen, Kreislauferkrankungen, Lungenerkrankungen, Infektionen wie beispielsweise Hepatitis oder HIV, Allergien, neurologisches wie beispielsweise Epilepsie und die Blutgruppe. In einem weiteren Feld könnten Variablen gespeichert sein wie Laborergebnisse, letzte Untersuchungen, Neoplasien, Röntgenergebnisse beispielsweise des Thorax, Medikamente und die letzte Verschreibung, Operationen, Schwangerschaft, Implantate wie beispielsweise Silikon und beispielsweise den Zahnstatus, also ob beispielsweise Prothesen vorliegen. In einem weiteren RAM- Feld könnte ein Rezept von einem Arzt eingegeben werden. Der Patient kann dann das Speicherelement zu einer Apotheke bringen, in der Einblick in die Stammdaten des Patienten genommen werden können und in das Rezeptfeld.

Vorzugsweise ist der einer zweiten Zugangsberechtigung zugeordnete zweite Speicherbereich mit einer ersten Zugangsberechtigung für den zugeordneten ersten Speicherbereich beschreibbar und mit der zweiten Zugangsberechtigung lesbar und löschbar. Ferner ist ein weiterer Speicherbereich, wie bspw. der Rezeptbereich, vorgesehen, der ohne Zugangsberechtigung lesbar ist. Hierbei bedeutet ohne Zugangsberechtigung insbesondere die übliche und normale Verwendung eines Kartenlesegerätes durch das Hilfspersonal eines Arztes in einer Arztpraxis, wobei bspw. auf ein ROM zugegriffen wird, in dem die Stammdaten des Patienten bspw. gespeichert sind.

Vorzugsweise sind die zwei Speicherbereiche mit Zugangsberechtigung auf wenigstens einem RAM angeordnet und/oder der Speicherbereich, der ohne Zugangsberechtigung lesbar ist, ist ein ROM.

Vorzugsweise ist das Speicherelement einstückig. Wenn vorzugsweise das Speicherelement auf einem flächigen Gebilde, insbesondere auf einer Karte angeordnet ist, kann eine übliche Versicherungskarte einer Krankenversicherung Verwendung finden. Die Karte ist hierbei insbesondere vorzugsweise eine sogenannte Chipkarte, in Form einer Kreditkarte oder einer "Smart Card".

Wenn vorzugsweise der Speicherinhalt der wenigstens zwei Speicherbereiche mit unterschiedlichen Verschlüsselungen versehen ist, ist es möglich verschiedenen Personen verschiedene Schlüssel zu geben, um den jeweiligen Personen nur ganz gewisse Bereiche auf dem Speicherelement zugänglich zu machen.

Die Aufgabe wird ferner durch ein System zur Erfassung und Speicherung personenspezifischer Daten umfassend einen Computer, ein Speicherelement wie vorstehend beschrieben und eine Verbindungseinrichtung, gelöst, wobei das Speicherelement mit der Verbindungseinrichtung derart in Eingriff bringbar ist, daß Daten von dem Speicherelement in den Computer einlesbar sind und/oder von dem Computer auf das Speicherelement schreibbar sind.

Ein derartiges System eignet sich vorzugsweise im Notfall beispielsweise wenn eine Person Unfallopfer geworden ist. Bei Ankommen des Arztes am Unfallort muß dieser lediglich die Versichertenkarte des Unfallopfers zu sich nehmen und in Eingriff mit der Verbindungseinrichtung bringen, um so auf die entsprechenden relevanten Daten zugreifen zu können.

Wenn vorzugsweise ein Zugangselement vorgesehen ist, mittels dem Daten zum Computer und/oder über den Computer zur Verbindungseinrichtung übermittelbar sind, wobei die Daten wenigstens einen Teil der Zugangsberechtigung aufweisen, ist es möglich, daß nicht jede Person sämtliche Daten von dem Speicherelement lesen kann, sondern nur diejenigen die die entsprechende Zugangsberechtigung bzw. das entsprechende Zugangselement haben. Das Zugangselement kann hierbei beispielsweise ein Speicherelement auf einer Karte sein, die der Arzt immer mit sich trägt. Auf dieser Karte können dann ein Teil der Zugangsberechtigungen gespeichert sein. Vorzugsweise ist die Karte noch mit einem PIN-Code abgesichert, so daß bei Verlust einer entsprechenden Zugangskarte eines Arztes diese nicht durch andere unberechtigte Personen verwendet werden kann. Der PIN-Code ist ein Beispiel für einen vorzugsweisen weiteren Teil der Zugangsberechtigung, die über ein Kommunikationsmittel in Form von Daten dem Computer und/oder über den Computer zur Verbindungseinrichtung übermittelbar ist.

Hilfspersonal bei einem Arzt kann beispielsweise nur den ROM- Bereich des Speicherelements lesen. Hierzu kann ein Zugangselement ein Code sein, der auf einem Computer gespeichert ist und beim Lesen der Karte dieser zugeführt wird, um so einen dem Zugangscode des Zugangselementes zugeordneten Bereich zu öffnen. Im Rahmen dieser Erfindung kann ein derartiger Computer auch ein Kartenlesegerät sein. Ein weiterer Bereich, nämlich beispielsweise ein Bereich eines RAM's ist beispielsweise für den Arzt allein vorgesehen. Dieser kann mit einem entsprechenden Zugangselement wie beispielsweise einem in seinem Computer befindlichen Code und einem persönlichen Code, insbesondere ein Strichcode oder biometrischer Code, den der Arzt in sein Computer eingeben muß gelesen und/oder beschrieben werden. Ein Zugangselement kann allerdings auch beispielsweise ein Speicherelement sein, daß auf einer transportierbaren Karte angebracht ist. Dem Arzt ist es natürlich vorzugsweise möglich auch den ROM- Bereich zu lesen. Ein Apotheker kann nur einen Teil des RAM's lesen und entsprechend löschen, nämlich den Teil, der für das Rezept vorgesehen ist. Hierzu ist ein Zugangselement vorgesehen, daß beispielsweise ein Code sein kann, der im Kartenleser oder Computer in der Apotheke vorhanden ist und über eine Schnittstelle dem Speicherelement der Versichertenkarte zugeführt werden kann, um so diesen Bereich für den Apotheker zugänglich zu machen.

Vorzugsweise sind Mittel zur Datenfernübertragung, insbesondere umfassend ein mobiles Telefon, vorgesehen. Mit diesen Mitteln zur Datenfernübertragung ist es möglich, die personenrelevanten Daten beispielsweise einem Krankenhaus zu übermitteln, um in dem Krankenhaus schon sämtliche Vorkehrungen für das Eintreffen eines Unfallopfers vorbereiten zu können.

Die Erfindung wird ferner durch ein System zur Erfassung und Speicherung personenspezifischer Daten, umfassend einen ersten Computer, der verschiedenen Speicherbereiche auf einem Computerspeicherelement beinhaltet, gelöst, wobei den verschiedenen Speicherbereichen wenigstens teilweise unterschiedliche Zugangsberechtigungen zugeordnet sind, einem tragbaren Speicherelement, auf dem Daten speicherbar sind, und einer Verbindungseinrichtung, wobei das tragbaren Speicherelement mit der Verbindungseinrichtung derart in Eingriff bringbar ist, daß Daten aus dem tragbaren Speicherelement in den ersten Computer übertragbar sind, wobei mittels Eingabe oder vorliegender entsprechenden Zugangsberechtigung die personenspezifischen Daten aus einem der Zugangsberechtigung entsprechenden Bereich des Computerspeicherelements lesbar und/oder veränderbar sind.

Durch diese erfindungsgemäße Lösung, bei dem der erste Computer verschiedene Speicherbereiche auf einem Computerspeicherelement aufweist, wobei die Speicherbereiche wenigstens teilweise unterschiedliche Zugangsberechtigungen umfassen, ist es möglich die personenspezifischen Daten auf einem schnellen Computersystem zwischenzuspeichern und von diesem Computersystem verschiedenen Personen, die unterschiedliche Zugangsberechtigungen haben, auch nur die jeweiligen Bereiche der Daten zugänglich zu machen.

Wenn vorzugsweise Mittel zur Datenfernübertragung vorgesehen sind, wobei die Daten aus dem ersten Computer einem weiteren der Verbindungseinrichtung zugeordnetem zweiten Computer übermittelbar sind, ist es nicht nötig, daß sämtliche Daten auf dem tragbaren Speicherelement gespeichert sind. Es ist möglich auf einem zentralen Computer, insbesondere einem Server, dem ersten Computer, weitere Daten zu speichern, was insbesondere bei hohen Datenmengen von Vorteil ist. Es ist dann möglich mittels des tragbaren Speicherelements und den Zugangsberechtigungen bzw. den Zugangsberechtigungselementen oder Codes einen Zugriff auf entsprechende Speicherbereiche des Computerspeicherelements auf dem ersten Computer zu ermöglichen. Wenn beispielsweise bei einem Unfall die Versichertenkarte eines Patienten eingelesen wird, werden diese Daten zusammen mit einem Code des Arztes dem ersten Computer beim Krankenhaus übermittelt, so daß die für diese Zugangsberechtigung zugänglichen Daten dem Arzt am Unfallort zurückübermittelt werden können, die dieser dann an seinem portablen zweiten Computer und/oder einem mobilen Telefon, das insbesondere Funktionalitäten eines Computers umfaßt, lesen kann und ggf. auch verändern kann.

Wenn vorzugsweise mittels der Verbindungseinrichtung zum Erkennen der Daten auf dem tragbaren Speicherelement ein elektromagnetisches Signal aussendbar ist und empfangbar ist, wobei das tragbare Speicherelement einen Transponder umfaßt, ist es nicht nötig, daß beispielsweise bei einem Unfall die Versichertenkarten überhaupt gefunden werden müssen. Es reicht, in die Nähe der jeweiligen Versichertenkarte zu gelangen, um die entsprechenden Daten von der Versichertenkarte in beispielsweise einen Computer des behandelnden Arztes zu übermitteln. Es wird beispielsweise ein Scanner mit elektromagnetischen Strahlen verwendet, mittels dem die auf dem Chip befindlichen Daten lesbar sind. Hierdurch wird in einem Unfall viel Zeit gespart. Eine derartige vom Patienten mit sich geführte Karte umfaßt vorzugsweise einen so genannten Aktiv- und/oder Passiv-Controller. Diese Technik ermöglicht problemloses Feststellen des Vorhanden- oder Nichtvorhandenseins eben dieser Patientenkarte beim Patienten. Der Controller besteht insbesondere aus einem kleinen Programm, das mit Hilfe einer Spule, mittels einer durch einen Träger gestellte, elektromagnetische, Energie initialisiert wird. Der initialisierende Träger kann von einem hand-held Computer oder aber von einem Notebook mit kleinem Zusatzgerät gestellt werden. Der Träger initialisiert die Spule, woraufhin der auf der Karte verankerte Chipsatz mit Energie versorgt wird. Dieser wiederum liest nun, gesteuert nach seinem Programm, bestimmte Daten aus dem Speicher und stellt diese dem Netzwerk zur Übertragung zur Verfügung. Es können Netzwerke wie das Ethernet mit langsamer Geschwindigkeit entsprechend der Norm IEE802.X Anwendung finden. Der Hand-held kann über eine übliche serielle Schnittstelle mit dem, die Daten verarbeitende Computer, zum Zwecke der Weiterleitung, im Falle der Notfallmaßnahme vor Ort, dem Notebook mit DFÜ Anschluß und entsprechende Protokollen verbunden werden. Dieses ist auch im Bereich der Veterinärmedizin anwendbar.

Der Vorteil dieser Technik liegt nun darin, daß gerade in Fällen größerem Unfallopferaufkommens, vom Autobahnunfall bis zum Tunnelbrand, die einerseits lebenswichtige und rettende Zeit der Befragung und vielleicht Suche nach Daten extrem reduziert wird und somit andererseits das Rettungspersonal wesentlich effektiver arbeiten kann, was zu erheblichen Kosteneinsparungen führt. Hier nicht zu vernachlässigen sind die erheblich reduzierten Folgen von unbekannten und unerkannten Vorschädigungen, die ohne Weitergabe der Daten auf dem Speicherelement mit passivem und/oder aktivem Controller so nicht möglich sind.

Die Erfindung wird ferner durch ein Computerprogrammprodukt und/oder ein Verfahren zum Beschreiben und/oder Lesen von Daten eines ersten Speicherelements wie vorgeschrieben gelöst, wobei zum Lesen und/oder Beschreiben von Daten aus wenigstens einem Speicherbereich des ersten Speicherelements, die wenigstens teilweise durch wenigstens eine Zugangsberechtigung gesichert sind, eine Zugangsberechtigung einem Speicherbereich eines zweiten Speicherelements zugeführt wird und mit wenigstens einer auf dem ersten Speicherelement- vorgebbaren Zugangsberechtigung verglichen wird, wobei bei Übereinstimmung der Zugangsberechtigung mit wenigstens einer der vorgebbaren Zugangsberechtigungen die Speicherbereiche des ersten Speicherelements, die dieser Zugangsberechtigung zugeordnet sind, freigegeben wird.

Durch das erfindungsgemäße Computerprogrammprodukt und das erfindungsgemäße Verfahren ist es möglich, je nach Zugangsberechtigung entsprechende Speicherbereiche des ersten Speicherelements freizugeben. Verschiedene Zugangsberechtigungen kommen beispielsweise für einen Arzt, für eine Arzthelferin und für die Apotheke in Frage. Der Arzt hat hierzu beispielsweise eine weitere Karte, auf der wenigstens ein Teil der Zugangsberechtigung beinhaltet ist. Diese weitere Karte muß beispielsweise einem Kartenlesegerät oder einem Computer eingefügt werden, um die darauf befindliche Zugangsberechtigung bzw. den entsprechenden Code einzulesen. Der Arzt gibt dann einen weiteren Code, wie beispielsweise einen PIN-Code, ein, um eine Zugangsberechtigung auf entsprechende Bereiche des ersten Speicherelements zu erhalten. Alternativ können auch biometrische Daten vom Arzt erfaßt und eingelesen werden, um eine Zugangsberechtigung zu erhalten.

Vorzugsweise werden zum Darstellen der freigegebenen Daten die Daten aus dem ersten Speicherelement in einen weiteren Speicherbereich des zweiten Speicherelements gelesen und einer Anzeigeeinheit zugeführt. Vorzugsweise werden zum Beschreiben des freigegebenen Speicherbereichs des ersten Speicherelements Daten in das zweite Speicherelement eingegeben und von dort auf das erste Speicherelement in den freigegebenen Speicherbereich übertragen und gespeichert. Wenn vorzugsweise eine Datenfernübertragung vorgesehen ist, kann das erste Speicherelement an einem anderen Ort sein als beispielsweise das zweite Speicherelement.

Erfindungsgemäß wird ein Speicherelement, das auf einer Karte angeordnet ist zur Speicherung und/oder Änderung von personenspezifischen Daten im Bereich des Gesundheitswesens und insbesondere im Bereich der Notfallmedizin zur schnellen Erfassung der Anamnese von Unfallpatienten verwendet.

Eine weitere Lösung der Aufgabe besteht bei einem Verfahren zur Rettung und/oder medizinischen Versorgung von Lebewesen im Notfall, insbesondere verunfallter Menschen, darin, daß eine Zentraleinheit über, insbesondere aktuelle, Daten über mobile Rettungsobjekte, insbesondere Rettungsärzte, Rettungssanitäter, Notärzte, Notarztwagen und/oder Rettungstransportwagen, verfügt und nach Meldung eines Notfallereignisses mit Lebewesen an die Zentraleinheit anhand der dem Notfallereignis zugrunde liegenden Daten und/oder anhand der Daten über die mobilen Rettungsobjekte die Zentraleinheit ein geeignetes mobiles Rettungsobjekt dem Notfallereignis zuordnet und eine Mitteilung über das Notfallereignis an das wenigstens eine zugeordnete mobile Rettungsobjekt sendet. Entsprechend dem Schadensort und/oder der Schadensart des Unfalls wird automatisch das augenblicklich am besten geeignete Rettungsfahrzeug und/oder das am besten geeignete Rettungspersonal ermittelt.

Um eine optimale Versorgung von Unfallopfern oder Menschen im Notfall zu gewährleisten, ist es erforderlich, daß die Zentraleinheit qualifizierte Daten über die Rettungsfahrzeuge und/oder das Rettungspersonal besitzt. Bei der Bestimmung der Rettungsobjekte können zusätzlich bereits vorhandene Angaben über das- Unfall- oder Notfallgeschehen berücksichtigt werden. Die Meldung des Ereignisses kann bspw. telefonisch bei einer Rettungsleitstelle, die z.B. über die bekannten Notrufnummern zu erreichen ist, erfolgen, wobei die Rettungsleitstelle mit der Zentraleinheit permanent oder nach Eingang der Unfallmeldung in Verbindung steht. In der Zentraleinheit können Unfälle oder Notfälle sowohl regional bzw. überregional als auch national bzw. überstaatlich zusammengefaßt und verwaltet oder überwacht oder gesteuert werden. Hierzu dient vornehmlich ein Zentralrechner bzw. ein Netz von Rechnern, die nach Eingang von Unfallereignissen sowohl lokal bzw. regional als auch (inter-)national für das jeweilige Unfall- bzw. Notfallereignis eine optimale Zuordnung von Rettungsobjekten vom jeweiligen Ereignis vornehmen.

Im Rahmen der Erfindung bedeutet die Meldung eines Notfallereignisses insbesondere die Übertragung von für das Notfallereignis spezifischen Daten. Diese Daten können schematisiert von einer einen Telefonanruf entgegennehmenden Person in ein Computersystem eingegeben und an die Zentraleinheit weitergeleitet werden. Die Daten können auch automatisch durch bspw. eine sprachgesteuerte Benutzerführung seitens des Anrufenden gemeldet werden.

Um eine optimale Versorgung der z.B. verunfallten Personen zu gewährleisten, ist es vorteilhaft, wenn die Daten über die mobilen Rettungsobjekte Daten über die Verfügbarkeit und/oder den Standort und/oder die Qualifikation aufweisen und an die Zentraleinheit übermittelt werden.

Die Übermittlung geschieht vorzugsweise bei jeder Änderung bzgl. der Daten über die mobilen Rettungsobjekte. Zur Sicherheit kann vorgesehen sein, nach vorgebbaren Zeitabständen die Daten über die mobilen Rettungsobjekte wenigstens teilweise an die Zentraleinheit zu übermitteln.

In einer Weiterbildung des Verfahrens werden die Daten und/oder der Standort eines mobilen Rettungsobjekts mittels GPS-Unterstützung an die Zentraleinheit übermittelt. Hierdurch ist es möglich, daß die Zentraleinheit durch Ausnutzung des Global Positioning-Systems (GPS) über Angaben verfügt, wo sich das momentan verfügbare Rettungsobjekt befindet. Somit kann stets herausgefunden werden, welches Rettungsobjekt z.B. ein Notarzt, sich in der Nähe des Notfallereignisses aufhält. Unter dem Begriff "Qualifikation eines Rettungsobjekts" kann z.B. bei dem verfügbaren Rettungspersonal die Ausbildung und die berufliche Qualifikation verstanden werden. Bei Fahrzeugen umfaßt die Qualifikation bspw. die Kapazität und die Ausrüstung bzw. Ausstattung des Wagens.

Eine weitere Verbesserung in der Rettung von verunglückten Menschen wird erreicht, wenn die Daten über die mobilen Rettungsobjekte, vorzugsweise in vorbestimmten Zeitabständen, aktualisiert werden.

Ist ein entsprechendes mobiles Rettungsobjekt, wie bspw. ein Arzt mit einer geringeren Qualifikation als dem Notfallarzt, am Not- oder Unfallereignis bereits vorhanden, so kann zur Verstärkung und Verbesserung des Rettungspersonals vorzugsweise eine Anforderungsanmeldung eines weiteren mobilen Rettungsobjekts an die Zentraleinheit übermittelt werden und die Zentraleinheit anhand der Anforderungsanmeldung und/oder anhand der Daten von verfügbaren mobilen Rettungsobjekten wenigstens ein weiteres mobiles Rettungsobjekt dem Ereignis zuordnen und eine Mitteilung an das weitere mobile Rettungsobjekt senden.

Ferner ist es vorteilhaft, wenn nach der Mitteilung des Notfallereignisses an das mobile Rettungsobjekt das mobile Rettungsobjekt eine Abmeldung an die Zentraleinheit sendet. Somit verfügt die Zentraleinheit über ein Wissen von verfügbaren und nicht verfügbaren Rettungsobjekten, d.h. von Objekten, die im Einsatz oder frei sind.

In einer vorteilhaften Weiterbildung der erfindungsgemäßen Lösung ist vorgesehen, daß die Zentraleinheit mit stationären Rettungseinrichtungen verbunden ist und über, insbesondere aktuelle, Daten der stationären Rettungseinrichtungen verfügt. Hierdurch wird in der Zentraleinheit ein Wissen über die verfügbaren Ressourcen von bspw. Krankenhäusern und Spezialkliniken gespeichert, so daß im allgemeinen stets in der Zentraleinheit bekannt ist, welche medizinischen Maßnahmen und Einrichtungen (z.B. in einer Spezialklinik für einen aktuellen Notfall) vorhanden sind und ob bspw. überhaupt ein Bett frei ist. Im Rahmen dieser Erfindung bedeutet eine Verbindung mit stationären Rettungseinrichtungen insbesondere eine Verbindung mit jeweils einem Datenverarbeitungssystem bspw. einem Computer in den Rettungseinrichtungen, auf denen relevante Daten zu den Rettungseinrichtungen gespeichert und aktualisiert werden.

Eine Qualitätssteigerung in der Rettung läßt sich ferner erreichen, wenn bei Meldung eines Weiterbehandlungsbedarfs des Lebewesens im Notfall vom mobilen Rettungsobjekt an die Zentraleinheit anhand des Weiterbehandlungsbedarfs und/oder anhand der Daten über die stationären Rettungseinrichtungen eine geeignete Rettungseinrichtung ermittelt wird und dem mobilen Rettungsobjekt Daten zu der ermittelten Rettungseinrichtung übermittelt wird. Der Weiterbehandlungsbedarf wird dadurch ermittelt, daß bspw. ein Notarzt über eine Datenleitung z.B. per Mobilfunk Angaben über die verunfallten Personen an die Zentraleinheit übermittelt. Hierzu kann ein standardisiertes Protokoll verwendet werden, in dem der Notarzt seine Diagnose mitteilt.

Die Daten zu der ermittelten Rettungseinrichtung können bspw. den Ort der Rettungseinrichtung umfassen. Es können bspw. die Koordinaten und die Adresse übermittelt werden. Die Koordinaten können dann in einem mobilen Rettungsobjekt wie einem Krankenwagen in das Navigationssystem eingespeist werden, um eine sofort beginnende Navigation automatisch beginnen zu lassen.

Außerdem ist es vorteilhaft, wenn während und/oder nach der Versorgung des verunfallten Lebewesens ein Datenaustausch, insbesondere von Patientendaten und/oder zur Weiterbehandlung, zwischen dem mobilen Rettungsobjekt und/oder der Zentraleinheit erfolgt. Durch diesen Datentransfer können schnell und umgehend zu einer verunfallten Person Daten aus anderen medizinischen Rechnernetzen gewonnen werden und einem Spezialisten in einer ausgewählten Notfallklinik zur Verfügung gestellt werden, während der Patient sich noch auf dem Wege zur Notfallklinik befindet. Hierdurch kann wertvolle Zeit zur Rettung des Menschen gewonnen werden, da der Spezialist sich während des Rettungstransports gezielt informieren und vorbereiten kann.

Im Rahmen dieser Erfindung umfaßt ein mobiles Rettungsobjekt einen Computer, auf dem die tatsächlichen Daten zu dem Notfallereignis speicherbar sind und mittels dem diese Daten einer Datenfernübertragungsvorrichtung zugeführt werden, um mit der Zentraleinheit zu kommunizieren.

Um eine geeignete stationäre Rettungseinheit entsprechend den Weiterbehandlungsanforderungen der zu rettenden Person auszuwählen, ist es von Vorteil, wenn die Daten über die stationären Rettungseinrichtungen Daten zur Verfügbarkeit und/oder Qualifikation und/oder Erreichbarkeit der Rettungseinrichtung aufweisen und der Zentraleinheit übermittelt werden.

Eine weitere Verbesserung in der medizinischen Versorgung ergibt sich dadurch, daß die Daten über die stationären Rettungseinrichtungen, vorzugsweise in vorbestimmten Zeitabständen und/oder nach einer Änderung der Daten bzw. des Status, aktualisiert werden. Somit kann zeitnah zu einem Unfallhergang sehr schnell und spontan auf den augenblicklichen Bedarf infolge des Unfallereignisses reagiert werden. Bspw. wenn ein Bett in einem Krankenhaus frei wird, wird dies umgehend an den Zentralrechner bzw. die Zentraleinheit weitergeleitet, so daß das freigemeldete Bett zur Verfügung steht.

Ferner kann sich bspw. ein Arzt über den zu behandelnden Patienten vor dessen Einlieferung informieren, wenn Daten zum Unfallereignis und/oder zum verunfallten Lebewesen (Mensch oder Tier) an die stationäre Rettungseinrichtung übermittelt werden.

Um sehr zeitnah zum Unfallereignis in Kliniken und Notfallkliniken auf das Unfallgeschehen zu reagieren und Maßnahmen zu ergreifen, ist es in einer Weiterbildung der Erfindung von Vorteil, wenn die Daten während der Versorgung des Lebewesens und/oder während des Transports zur zugeordneten stationären Rettungseinrichtung übermittelt werden.

Als weitere Lösung der Aufgabe wird ein Computerprogramm mit Programmcode zur Durchführung des oben beschriebenen Verfahrens zur Rettung und/oder medizinischen Versorgung von verunfallten Lebewesen, insbesondere Menschen oder Tieren, so daß die beschriebenen Verfahrensschritte ausgeführt werden können, wenn das Programm in wenigstens einem Computer ausgeführt wird, angegeben.

Das Computerprogramm ist mittels eines Systems zur Rettung von Lebewesen ausführbar, wobei das System mindestens eine Zentraleinheit, ein mobiles Rettungsobjekt und eine stationäre Rettungseinrichtung aufweist. Die Zentraleinheit und die Rettungsobjekte sind vorzugsweise über eine Funkdatenleitung miteinander in Verbindung. Die Verbindung zwischen Zentraleinheit und Rettungseinrichtung kann über eine Datenfernleitung realisiert werden.

Ferner ist ein Computerprogramm mit Programmcode, der auf einem maschinenlesbaren Träger gespeichert ist, zur Durchführung des oben beschriebenen erfindungsgemäßen Verfahrens zur Rettung und/oder medizinischen Versorgung von Lebewesen, so daß die beschriebenen Verfahrensschritte ausgeführt werden können, wenn das Programm in einem Computer ausgeführt wird, vorgesehen.

Die Erfindung wird nachstehend ohne Beschränkung des allgemeinen Erfindungsgedankens anhand von Ausführungsbeispielen unter Bezugnahme auf die Zeichnungen beschrieben, auf die im übrigen bezüglich aller im Text nicht näher erläuterten erfindungsgemäßen Einzelheiten ausdrücklich verwiesen wird. Es zeigen:
- Fig. 1: eine schematische Darstellung eines erfindungsgemäßen Systems zur Erfassung und Speicherung personenspezifischer Daten,
- Fig. 2: ein vereinfachtes Flußdiagramm eines Ablaufs in einer Arztpraxis,
- Fig. 3: ein vereinfachtes Flußdiagramm eines speziellen Teils eines Verfahrensablaufs in einer Arztpraxis,
- Fig. 4: ein vereinfachtes Flußdiagramm für einen Verfahrensablauf in einer Apotheke,
- Fig. 5: eine schematische Darstellung eines weiteren Ausführungsbeispiels eines Systems zur Erfassung und Speicherung personenspezifischer Daten und/oder Daten von Tieren und
- Fig. 6: ein vereinfachtes Ablaufschema eines Verfahrens zur Rettung von verunfallten Menschen oder Tieren.

In den folgenden Figuren sind gleiche Elemente durch dieselben Bezugsziffern gekennzeichnet. Von einer erneuten Vorstellung der jeweiligen Elemente wird in diesen Fällen abgesehen.

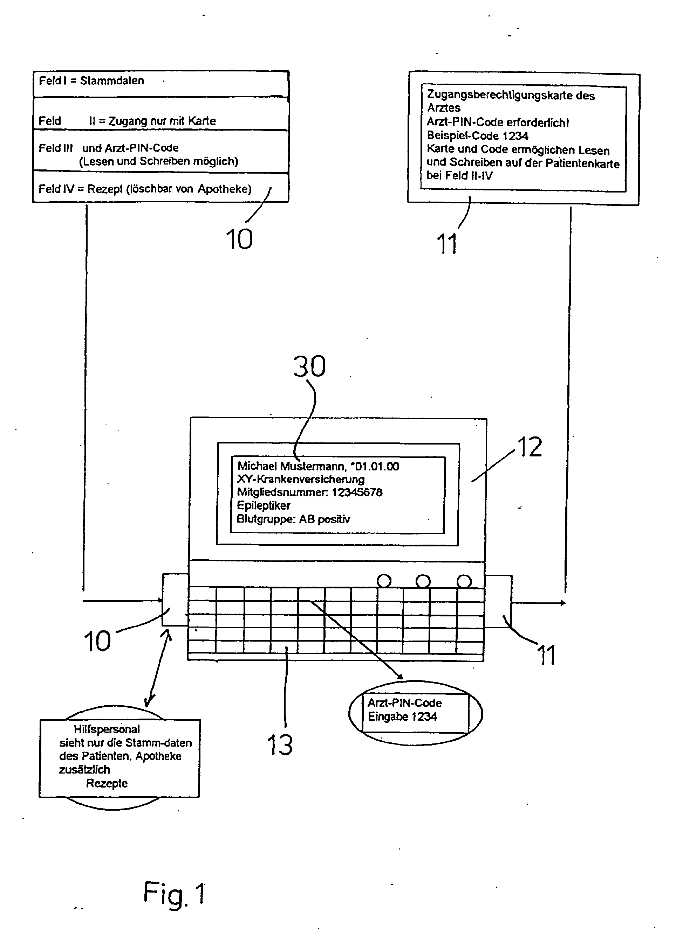

In Figur 1 ist eine schematische Darstellung eines Ausführungsbeispiels eines Systems zur Erfassung und Speicherung personenspezifischer Daten dargestellt. Eine Versichertenkarte 10 ist in 4 Felder aufgeteilt, nämlich dem Feld I, in dem Stammdaten gespeichert sind, dem Feld II, III und IV, in denen weiter Daten speicherbar sind. Das Feld I ist ohne weitere Zugangsberechtigung einlesbar und enthält Stammdaten wie vorstehend schon beschrieben wurde. Die Felder II und III sind nur mit einer Zugangsberechtigungskarte 11 und einem Arzt-PIN-Code zugänglich. Hier ist das Lesen des Speicherinhaltes möglich und das Beschreiben. Das Feld II enthält beispielsweise Daten zu Diagnosen und das Feld III enthält weitere Daten, wie vorstehend schon beschrieben wurde.

Das Feld IV kann beispielsweise den Inhalt eines Rezeptes aufweisen. Dieses Feld ist vom Arzt beschreibbar und auch wieder löschbar und in der Apotheke lesbar und löschbar.

In der Arztpraxis kommt nun ein Patient und übergibt seine Versichertenkarte an das Hilfspersonal. Diese führt die Versichertenkarte in ein Computer 12 ein. Das Personal beim Arzt kann dann die Stammdaten einlesen und am Monitor betrachten. Der Arzt selber hat in seinem Zimmer einen weiteren Computer 12 stehen, und steckt seine Zugangsberechtigungskarte in eine entsprechende Schnittstelle und gibt ferner dann einen PIN-Code ein. Die Zugangsberechtigungskarte zusammen mit dem PIN-Code ermöglichen das Lesen und Schreiben auf der Versichertenkarte in den Feldern II, III und IV. Die Codeeingabe erfolgt über die Tastatur 13. Die Daten können dann auf dem Monitor 30 angesehen werden.

Es ist natürlich möglich, daß mittels Datenleitungen die Daten der Versichertenkarte von dem Computer im Vorzimmer zu dem Computer beim Arzt transferiert werden können. Es ist somit nicht notwendig, daß die Versichertenkarte in einer weiteren Kartenlese- und Speichereinrichtung in den Computer des Arztes eingesteckt werden muß. Die Variante aus Figur 1, bei der sowohl die Versichertenkarte als auch die Zugangsberechtigungskarte des Arztes in jeweils verschiedene Lese- und Schreibschnittstellen eingeführt sind, ist allerdings bevorzugt.

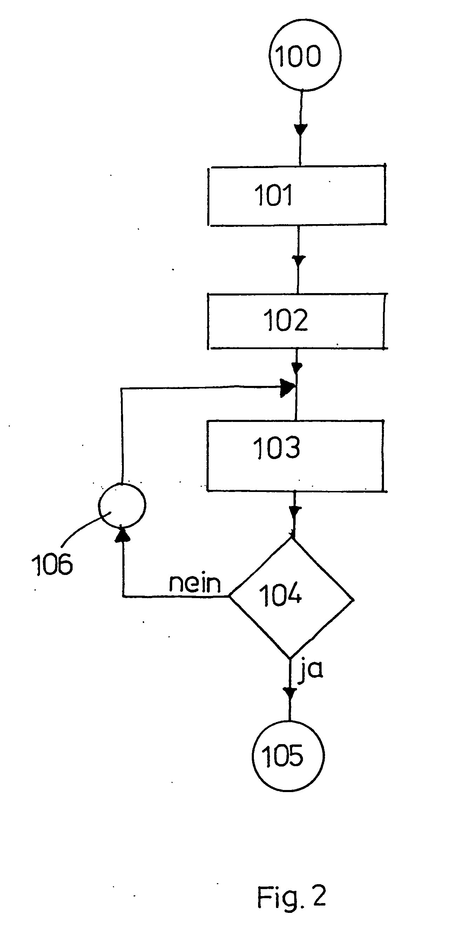

In Figur 2 ist ein Flußdiagramm mit einer vorzugsweise Verfahrensführung in der Arztpraxis dargestellt. Die Verfahrensführung beginnt bei 100 mit Start. Zunächst wird die Versichertenkarte in einen Computer 12 eingeführt (Schritt 101), dann werden die Stammdaten gelesen (Schritt 102). Als nächstes werden die Stammdaten auf dem Monitor angezeigt (Schritt 103), um diese Schritte auszuführen, werden übliche Computerprogramme verwendet. Hierzu werden Daten in an sich üblicher Weise in einen Prozessor eingelesen und in entsprechende Speicherbereiche auf dem Computer abgelegt und zur Ausgabe an den Monitor aufgearbeitet.

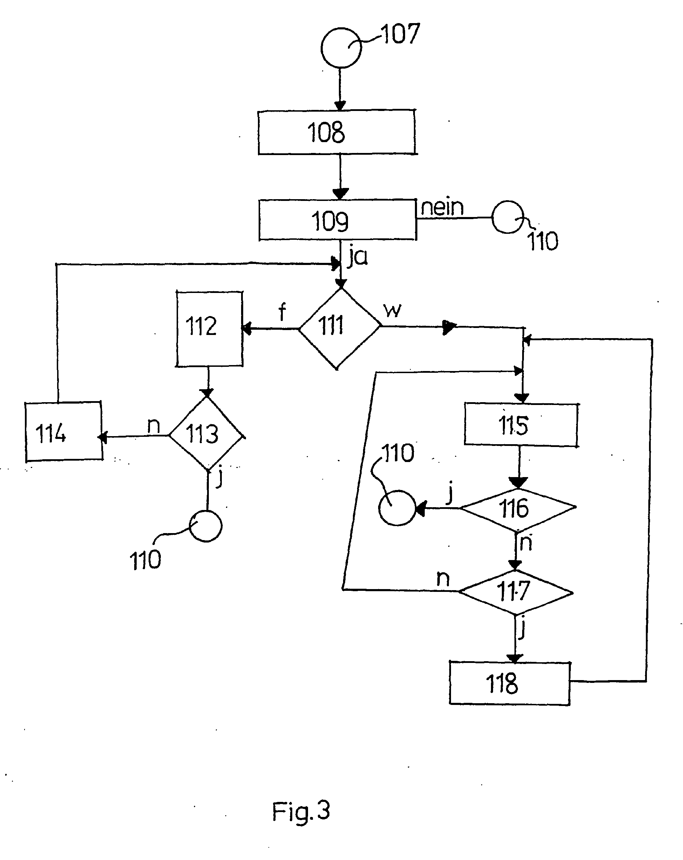

Bei (Schritt 104) erfolgt eine Abfrage, ob die Versichertenkarte entnommen wurde. Ist dieses der Fall endet der Verfahrensablauf gemäß Figur 2. Ist dieses nicht der Fall erfolgt eine erneute Abfrage bei 104. Es ist nun möglich im Feld 106 einen Einschub einzufügen. Dieser Einschub ist in einem vereinfachten Verfahrensablauf der Figur 3 näher beschrieben.

Mit 107 ist der Start des Einschubs beim Arzt dargestellt. Bei 108 wird die Zugangsberechtigungskarte in den Arztcomputer eingeführt. Bei 109 wird die Zugangsberechtigung geprüft. Weist die Zugangsberechtigungskarte keine Zugangsberechtigung auf, die mit einer entsprechenden auf den Computern oder in der Versichertenkarte 10 gespeicherten Zugangsberechtigung übereinstimmt, ist also im Falle von "nein" das Ende des Einschub "Arzt" bei 110 erreicht. Stimmt die Zugangsberechtigung mit einer der gespeicherten Zugangsberechtigungen überein ("ja"), wird in einem Feld 111 auf die Eingabe eines PIN-Codes über eine Tastatur gewartet und der eingegebene PIN-Code geprüft. Ist der eingegebene PIN-Code falsch (f) wird ein vorher auf Null gesetzter Zähler um eins erhöht (Schritt 112). Es erfolgt dann bei 113 die Abfrage, ob der Zähler größer als 3 ist. Wird diese Frage bejaht, endet der Einschub beim Arzt in 110. Wird diese Frage verneint (n) wird bei 114 eine Warnung auf dem Monitor angezeigt und es geht zurück zum Feld 111. Stimmt der eingegebene PIN-Code (w) geht das Verfahren weiter zum Feld 115, in der die weiteren Felder der Versichertenkarte gemäß der eingegebenen Zugangsberechtigung angezeigt werden. Im Feld 116 wird die Anfrage gestellt, ob die Zugangskarte entnommen wurde. Ist dieses der Fall (j) endet der Einschub bei 110. Ist dieses nicht der Fall (n), wird darauf gewartet, ob Daten eingegeben werden (Feld 117). Werden Daten nicht eingegeben, geht der Verfahrensablauf zurück zu Feld 115. Werden Daten eingegeben, werden im Feld 118 die Daten auf der Versichertenkarte geändert. Es geht danach zurück zum Feld 115.

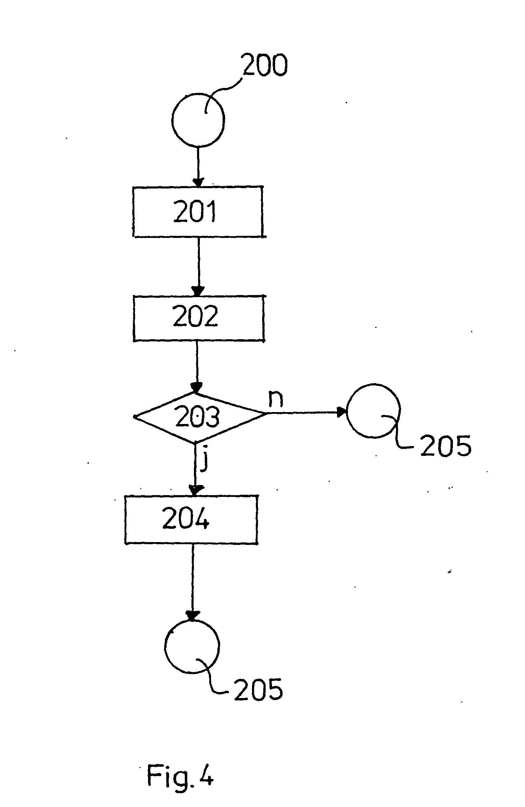

In Figur 4 ist ein Verfahrensablauf in beispielsweise einer Apotheke vereinfacht dargestellt. Bei 200 beginnt der Verfahrensablauf. Zunächst wird die Versichertenkarte in einen Kartenleser eingeführt (Feld 201). In Feld 202 wird die Zugangsberechtigung für den Rezeptbereich auf der Versichertenkarte zur Versichertenkarte übergeben (Feld 202). In Feld 203 wird die Frage gestellt, ob die Zugangsberechtigung in Ordnung ist. Ist diese nicht in Ordnung (n) endet das Verfahren in der Apotheke. Ist die Zugangsberechtigung in Ordnung (j) werden im Feld 204 die Daten des Rezeptteils angezeigt, ggf. die Daten dem Computersystem zur Abrechnung mit den Krankenkassen des Apothekers zugeführt und danach im Rezeptteil bzw. Feld IV der Versichertenkarte gelöscht. Anschließend endet das Verfahren bei der Apotheke.

Die vorstehend genannten Verfahrensabläufe gemäß den bevorzugten Ausführungsbeispielen und auch den weiteren Ausführungsbeispielen werden zum größten Teil mittels Computerprogrammschritten durchgeführt, die an sich für den Fachmann bekannt sind und somit ohne weiteres ausführbar sind und bedürfen an dieser Stelle keiner weiteren Erläuterung.

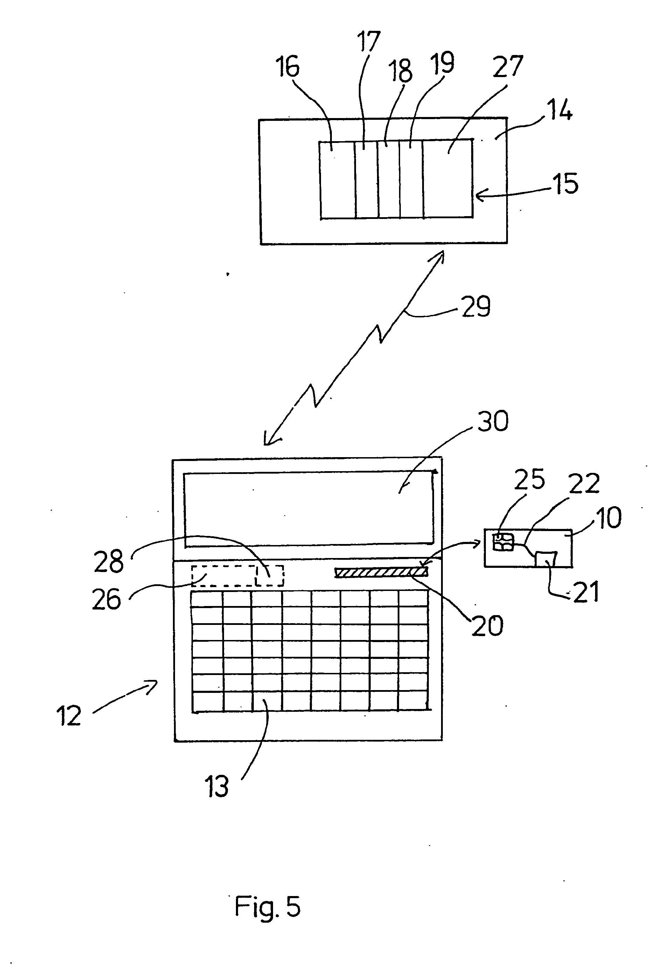

Figur 5 zeigt ein weiteres erfindungsgemäßes Ausführungsbeispiel in schematischer Darstellung. In diesem Ausführungsbeispiel sind auf einem externen Computer (14), der beispielsweise in einer Klinik lokalisiert sein kann in einem Computerspeicher Speicherbereiche 16, 17, 18 und 19 für patientenrelevante Daten vorgesehen. Der Speicherbereich 27, der hier auch nur schematisch dargestellt ist, soll beispielsweise ein Speicherbereich auf dem Prozessor oder einer Festplatte darstellen. Die verschiedenen Speicherbereiche 16 bis 19 sind für die verschiedenen Felder der Daten von Patienten vorgesehen.

Ein Unfallarzt hat beispielsweise einen Laptop oder einen Palm-Computer, also einen Computer (12) vor Ort. In diesem Computer (12) wird beispielsweise eine Versichertenkarte 10 in eine beispielsweise in den Computer integrierten Verbindungseinrichtung 20 eingeführt. Die in dem Speicher 25 der Versichertenkarte 10 gespeicherten Daten können so ggf. von dem Arzt übernommen werden. Die in dem Speicher 25 gespeicherten Daten sind hier beispielsweise nur Stammdaten. Ein in dem Computer 12 angeordneter schematisch dargestellter Speicher 26 umfaßt insbesondere einen Speicherbereich 28. Die Stammdaten werden nun beispielsweise auf dem Monitor 30 dargestellt. Mittels einer Datenübertragung 29 werden entsprechenden Daten dem Computer 14 per Funk beispielsweise übermittelt. Zudem ist in dem Speicherbereich 28 des Speichers 26 eine Zugangsberechtigung des Arztes gespeichert. Diese Zugangsberechtigungsdaten bzw. der Zugangsberechtigungscode wird über die Datenübertragung 29 dem Computer 14 zugeführt. Außerdem gibt der Notfallarzt beispielsweise einen PIN-Code auf der Tastatur 13 des Computer 12 ein. Dieser PIN-Code wird auch an den Computer 14 übertragen. Ist die gesamte Zugangsberechtigung, bestehend aus der auf dem Speicherbereich 28 gespeicherten Zugangsberechtigung und dem PIN-Code, korrekt, so kann der Arzt die Bereiche 16 bis 19 des Speichers 15 einsehen und ggf. auch verändern.

In diesem Ausführungsbeispiel sind also die wesentlichen personenrelevanten Daten auf einem Computer in beispielsweise einer Klinik gespeichert. Er kann allerdings auch ein Zentralcomputer sein, der beispielsweise für ein ganzes Land sämtlich Daten speichert.

Alternativ hierzu können in einem anderen Ausführungsbeispiel, das auch mittels Figur 5 beschrieben wird, sämtliche Daten auf der Versichertenkarte 10 im Speicher 25 gespeichert sein und zwar in den wie vorstehend beschriebenen verschiedenen Bereichen, die mittels verschiedenen Zugangsberechtigungen zugänglich sind. Mittels der Zugangsberechtigungsdaten im Speicherbereich 28 des Speichers 26 und dem PIN-Code, den der Arzt eingibt, können diese Daten dann entsprechend- gelesen werden.

Sofern die Versichertenkarte nicht am Unfallort gefunden werden kann, ist es vorteilhaft, die Versichertenkarte mit einem Transponder 21 zu versehen, der mit einer Verbindungsleitung 22 mit dem Speicher 25 verbunden ist. Der Laptop des Arztes umfaßt dann beispielsweise eine Art Funkgerät, das elektromagnetische Strahlen aussendet, die die auf dem Speicher 25 gespeicherten Daten lesbar machen können. Hierzu kann die Reichweite entsprechend begrenzt vorgesehen sein, um nicht die Daten verschiedener Versichertenkarten, die in einigen Metern Abstand zueinander sich befinden, durcheinander zu bringen. Das Funkgerät bzw. der Sende-/Empfänger kann auch in einem zusätzlichen Gerät zu dem Computer 12 vorgesehen sein und mit dem Computer mittels einer Datenverbindung verbunden sein. Die Versichertenkarte fungiert dann als eine Art ID-Tacker. Vorzugsweise werden hierzu über eine kleine Antenne Gigahertzwellen in ein Oberflächenwellenbauelement eingekoppelt. Die Oberflächenwellen erhalten durch den Leiter eine entsprechende Codierung. Die reflektierte Welle wird dann wieder limitiert bzw. ausgekoppelt und von einer Empfangsvorrichtung empfangen und weiterverarbeitet.

Es ist insbesondere vorzusehen, daß ein Arzt ohne Eingabe eines speziellen PIN-Codes der Versichertenkarte, die nur für eine Versichertenkarte gilt, an die Daten gelangt, da möglicherweise der Versicherte bzw. Patient in gewissen Momenten nicht ansprechbar ist. Der PIN-Code und die weiteren Zugangsberechtigungsdaten sollten somit unabhängig von der jeweiligen einzelnen Versichertenkarte funktionieren. Ferner ist es sinnvoll, auf der Versichertenkarte ein Foto des Patienten vorzusehen, um eine schnelle Identifikationsmöglichkeit zu geben.

Die Zugangskarte bzw. die Zugangsberechtigung, die auf einem Speicher in einem Computer gespeichert ist, sollte für die lese- und schreibberechtigten Personen mittels eines PIN-Codes gesichert sein, um Mißbrauch auszuschließen.

Eine Einbettung eines Paßbildes in Form von Daten und/oder auf der Versichertenkarte in optischer Art und Weise verhindert Mißbrauch der Karte bei Verlust oder Diebstahl. Die Beschreibbarkeit bietet den Vorteil der Vermeidung der Doppeldiagnostik, wodurch die damit verbundenen Kosten und entsprechenden medizinischen Risiken vermieden werden können. Zudem wird auf der Karte die letzte Medikation vorzugsweise dokumentiert, womit das Ärzte-Hopping wie auch die Doppelbehandlung durch Nichtwissen der Vorbehandlung beschränkt wird.

Der Datentransfer per Intranet oder Internet oder per Funk oder Telefon kann eine vorzugsweise Ausgestaltung sein.

Für den deutschen Bereich ist vorzugsweise ein Schnittstellenprotokoll wie beispielsweise eine Capi-Anbindung zur Nutzung eines pear-to-pear-Protokoll über ISDN unter Nutzung des TCP/IP-Stapels für schnellen Datentransport nutzbar. Ein entsprechender ISDN-Controller sollte mit der Controller-Software entsprechende B-Kanal-Protokolle in ISDN unterstützen. Diese sind beispielsweise X.75, HDLC transparent, transparent, X.25, ISO 8208 (X.25 DTE-DTE), X.31 case a/b, T.90, Fax G3, V.110 und V.120. Hieraus entsteht eine Kompatibilität zu gebräuchlichen Standards und Geräten und damit eine entsprechende Kostensenkung. Durch Einbeziehung eines Capi-Ports, der virtuelle Modems für Datenverbindungen mit X.32-X.75 Datenkompression während der Übertragung zur Verfügung stellt, sowie mittels eines Kompressionsverfahrens, in Konformität zur Capi 2.0-Spezification bzw. V.42bis (Capisoftcompression X.75/V.42bis), gelingt eine unkomplizierte Anbindung auch an analoge Netzwerke.

Ein direkter Zugriff auf medizinisch relevante Daten von Klinik zu Klinik, Notarztwagen zu Klinik, Arzt zu Klinik, Klinik zu Arzt u.s.w. erscheint sinnvoll. Dieses insbesondere bei akuten Einsätzen, bei Massenunfällen und im Katastrophenschutz. Eine Implementierung eines NDIS-WAN-Ports kann hierbei von Vorteil sein. Entsprechend ist es auch möglich mit UMTS bzw. GSM Verbindungen herzustellen. Die Datenfernübertragung zwischen den Computern des Arztes und bzw. des Kartenlesers des Arztes und einer Notfallklinik bzw. einem Krankenhaus kann über Mobilfunk stattfinden.

Ferner ist es möglich, auch automatisch ein medizinisches Protokoll auszufüllen, das dann auch vorzugsweise per Datenfernübertragung schon an die Krankenkassen bzw. an die Krankenhäuser übermittelt werden kann.

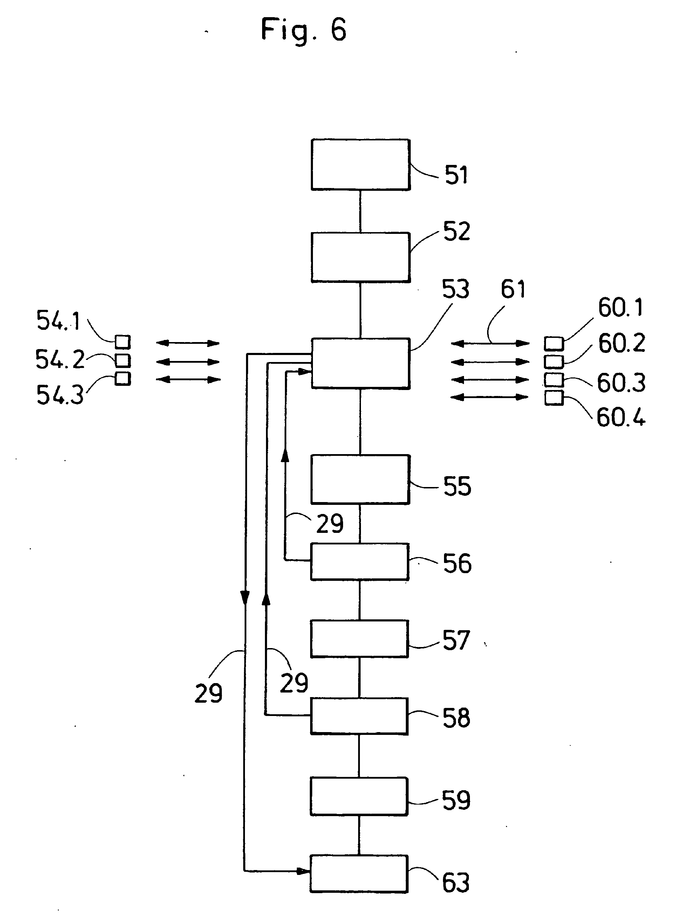

Fig. 6 zeigt ein vereinfachtes, schematisches Ausführungsbeispiel eines Verfahrens zur Rettung und/oder medizinischen Versorgung von (verunfallten) Lebewesen. Das Verfahren kann nicht nur zur Rettung von Menschen im Notfall, sondern auch zur Rettung von Tieren, insbesondere Pferden und Hunden, angewendet werden.

Nach Eintritt eines Unfalls oder Notfalls 51, bei dem Menschen und/oder dem Tier, das verletzt ist oder anderweitig zu Schaden gekommen ist, wird eine Meldung 52 des Unfalls 51 an einen Zentralrechner 53 -übermittelt. Der Unfall 51 kann bspw. telefonisch bei einer Rettungsleitstelle gemeldet werden mit Angaben über den Schadensort und/oder die Schadensart, so daß von der Rettungsleitstelle dann eine entsprechende Meldung 52 an den Zentralrechner 53 weitergeleitet wird. Die Rettungsleitstelle kann über eine entsprechende Datenfernleitung mit dem Zentralrechner permanent oder nur bei entsprechendem Eintritt eines Unfalls 51 mit dem Zentralrechner 53 verbunden sein.

Der Zentralrechner 53 ist darüber hinaus mit mobilen Rettungsobjekten 54.1, 54.2, 54.3 über Funkverbindung oder mobile Datenleitungen verbunden. Die mobilen Rettungsobjekte 54.1, 54.2, 54.3 sind über das sog. Global Position-System (GPS) ortbar, so daß der Zentralrechner 53 stets aktuell den Standort der mobilen Rettungsobjekte 54.1, 54.2, 54.3 abgespeichert hat. Darüber hinaus können auch weitere Daten der mobilen Rettungsobjekte 54.1, 54.2, 54.3 über die Ausstattung bzw. die Qualifikation bzw. die Art des Rettungsobjekts an die Zentraleinheit 53 übermittelt werden. Mobile Rettungsobjekte 54.1 bis 54.3 können bspw. Notärzte, Notarztwagen und Rettungstransportwagen sein.

Entsprechend den gemeldeten Angaben über das Unfallereignis 51 und anhand der Angaben der verfügbaren mobilen Rettungsobjekte 54.1, 54.2, 54.3 wird vom Zentralrechner 53 ein augenblicklich vorhandenes freies Rettungsobjekt in einem Ermittlungsschritt 55 festgelegt. Das entsprechend den Anforderungen des Unfallereignisses 51 zugeordnete Rettungsobjekt erhält eine Mitteilung über den Unfall 51 und wird zum Einsatzort bzw. zum Unfallgeschehen dirigiert. Da sich das zum Unfallereignis zugeordnete Rettungsobjekt sich im Einsatz befindet, wird eine Abmeldung 56 des Rettungsobjekts der -Zentraleinheit 53 mitgeteilt. Somit ist im Zentralrechner 53 gespeichert, daß das zum Einsatzort befehligte Rettungsobjekt für einen weiteren Einsatz an einem anderen Unfallort nicht (mehr) zur Verfügung steht.

Am Unfallort 51 eingetroffen, kann in einem ersten Schritt der medizinischen Erstversorgung der Unfallopfer die Versichertenkarte des Unfallopfers gescannt werden. Hierzu verfügt die Versichertenchipkarte über Merkmale, wie sie voranstehend für eine ebenfalls erfindungsgemäße Versicherungskarte bzw. Speicherelement beschrieben wurde. Darüber hinaus kann die Versichertenchipkarte mit einer Spule ausgerüstet sein, so daß insgesamt ein passiver Transponder ausgebildet wird.

Durch die Aufnahme der Patientendaten können diese Patientendaten in einen Rechner, z.B. Laptop, eingegeben werden, der eine Netzverbindung mit der Zentraleinheit 53, insbesondere über Funk, herstellen kann. Darüber hinaus findet in einem weiteren Schritt 58 die Erstversorgung der verunfallten Person statt, wobei der Notarzt die Weiterbehandlungsanforderungen in seinen Rechner eingibt. Diese Weiterbehandlungsanforderungen werden über die bereits genannte mobile Datenfernverbindung übermittelt, die mit dem Zentralrechner 53 besteht.

Der Zentralrechner 53 ist darüber hinaus auch mit Notfallkliniken und anderen Krankenhäusern 60.1 bis 60.4 über entsprechende Verbindungen 61 verbunden. Diese stationären Rettungseinrichtungen 60.1 bis 60.4 liefern ebenfalls Daten an den Rechner 53 über die momentane Belegung der Betten bzw. die augenblicklich vorhandenen Behandlungsressourcen. Darüber hinaus kann eine stationäre Rettungseinheit 60.1 bis 60.4 weitere Daten über die Qualifikation des Personals bzw. über spezielle Angebote und Geräte, wie Diagnose- und Behandlungsgeräte, der Klinik dem Zentralrechner 53 mitteilen. Diese Daten werden stets aktualisiert. Das bedeutet insbesondere, daß bei Freiwerden eines Bettes in einem Krankenhaus, dieses frei gewordene Bett dem Zentralrechner mitgeteilt wird.

Aus den Daten der verfügbaren stationären Rettungseinrichtungen 60.1 bis 60.4 und anhand des übermittelten Weiterbehandlungsprotokolls des Notarztes am Unfallgeschehen 51 ermittelt der Zentralrechner 53 in einem weiteren Schritt 59 eine entsprechende Notfallklinik, die eine optimale medizinische Versorgung der verunfallten Person gewährleistet. Bei der Auswahl der entsprechenden Notfallklinik durch den Zentralrechner 53 können weitere Faktoren wie z.B. der Ort, und die Qualifikation des Arztpersonals Einfluß haben.

Die vom Zentralrechner 53 ermittelte Notfallklinik wird dem mobilen Rettungsobjekt (Notarzt) am Einsatzort mitgeteilt bzw. zugewiesen (Verfahrensschritt 63). Gleichzeitig erfolgt eine Meldung des Unfallereignisses 51 und des Weiterbehandlungsbedarfs sowie einer Übertragung der Patientendaten des Unfallopfers an die Notfallklinik. Die Daten des zu behandelnden Patienten sowie die Anamnese und Verletzungen des Unfallopfers liegen damit in der Notfallklinik einem Ärzteteam schon vor, während der Patient noch vom Notarzt vor Ort am Unfallereignis 51 versorgt wird.

In einer Alternative kann bspw. der Notarzt am Unfallereignis 51 über die mobile Datenleitung einen weiteren Rettungswagen bspw. mit weitergehenden Gerätschaften anfordern, der entsprechend des Anforderungsprofils und der Verfügbarkeit des Rettungsobjektes vom Zentralrechner 53 bestimmt wird.

Die Organisation des aufgezeigten Rettungssystems mit einem Zentralrechner bzw. Zentraleinheit, mobilen Rettungsobjekten und stationären Rettungseinrichtungen, kann global wie auch lokal organisiert sein, wobei mehrere lokale Zentralrechner auf verschiedene Regionen und Städte verteilt sein können, wobei die Gesamtheit der Rechner die Zentraleinheit bildet. Hierdurch kann eine Region bezüglich der medizinischen Versorgung gut überwacht werden, so daß die Wartezeiten und Anlieferungszeiten von Unfallpersonen mittels des aufgezeigten Verfahrens verkürzt werden können. Hierdurch können mehr Menschen schneller und besser medizinisch versorgt werden, so daß eine Steigerung in der Rettung von Menschen ermöglicht wird. Aufgrund der Überwachung von Regionen läßt sich eine Optimierung des Rettungswesens auf lokalen wie gebietsüberschreitenden Ebenen erreichen. Es kann insbesondere durch die Überwachung bzw. die Auswertung der Daten eine Statistik angefertigt werden, mittels der der Bedarf an mobilen Rettungsobjekten und stationären Rettungseinrichtungen sowohl bzgl. der Qualität (insbesondere Qualifikation von Ärzten und Güte der Betten und Gerätschaften, wie insbesondere bspw. auf Herz-Kreislauf-Erkrankungen optimierte Betten mit zugehörigen medizinischen Geräten und Personal in den Krankenhäusern) als auch Quantität (bspw. der Anzahl der benötigten Rettungswagen und/oder Notfallwagen in der Stadt Kiel, Anzahl der benötigten Rettungssanitäter und/oder Notärzte, Anzahl der benötigten Betten etc.) insbesondere für vorgebbare Zeiträume bestimmt werden. Die Rettungsobjekte und Rettungseinrichtungen können dann entsprechend optimiert werden, was auch zu Kosteneinsparungen führt.

Im Rahmen dieser Erfindung umfaßt der Begriff personenspezifische Daten insbesondere auch tierspezifische Daten.

### Bezugszeichenliste

- 10: Versichertenkarte
- 11: Zugangsberechtigungskarte
- 12: Computer
- 13: Tastatur
- 14: Computer
- 15: Computerspeicher
- 16, 17, 18, 19,: Speicherbereich
- 20: Verbindungseinrichtung
- 21: Transponder
- 22: Verbindungsleitung
- 25: Speicher
- 26: Speicher
- 27: Speicherbereich
- 28: Speicherbereich
- 29: Datenübertragung
- 30: Monitor
- 51: Unfallereignis
- 52: Meldung (Unfallereignis)
- 53: Zentralrechner
- 54.1 bis 54.3: mobile Rettungsobjekte
- 55: Ermittlung des Rettungsobjekts
- 56: Abmeldung des Rettungsobjekts
- 57: Datenaufnahme des Patienten
- 58: Erstversorgung / Weiterbehandlungsbedarf
- 59: Ermittlung der Notfallklinik
- 60.1 bis 60.4: stationäre Rettungseinrichtungen
- 61: Verbindung
- 63: Einweisung / Zuweisung
- 100: Start
- 101: Einführen der Versichertenkarte
- 102: Lesen der Stammdaten
- 103: Anzeige der Stammdaten auf Monitor
- 104: Abfrage "Entnahme Versichertenkarte"
- 105: Ende
- 106: Einschubfeld
- 107: Start Einschub "Arzt"
- 108: Einführen Zugangskarte
- 109: Prüfen Zugangsberechtigung
- 110: Ende Einschub "Arzt"
- 111: Warten auf PIN-Code und Prüfen PIN-Code
- 112: Zähler
- 113: Zähler > 3?
- 114: Warnungsanzeige
- 115: Anzeige weiterer Felder
- 116: Entnahme Zugangskarte ?
- 117: Eingabe Daten
- 118: Änderung der Daten auf Versichertenkarte
- 200: Start Apotheke
- 201: Einführen Versichertenkarte
- 202: Übergabe Zugangsberechtigung für Rezeptbereich
- 203: Zugangsberechtigung ok ?
- 204: Anzeige der Daten im Rezeptteil und Löschen der Daten im Rezeptteil
- 205: Ende "Apotheke"
- w :: wahr
- f :: falsch
- j :: ja
- n :: nein

## Patentansprüche

1. Speicherelement (25) zur Speicherung medizinischer personenspezifischer Daten mit wenigstens zwei Speicherbereichen, wobei wenigstens ein Speicherbereich ein ROM ist und wenigstens ein Speicherbereich ein RAM ist, wobei den Speicherbereichen unterschiedliche Zugangsberechtigungen zugeordnet sind, wobei das Speicherelement (25) einstückig ist, und wobei das Speicherelement (25) auf einem als Chipkarte ausgebildeten flächigen Gebilde (10) angeordnet ist,
**dadurch gekennzeichnet, daß**
das Speicherelement (25) in vier Speicherbereiche aufgeteilt ist,
wobei der erste Speicherbereich als ROM ausgebildet ist, in dem personenspezifische Stammdaten, insbesondere Name, Adresse, Daten der Krankenversicherung, Daten zum nächsten Angehörigen, Daten zum Hausarzt, biometrische Daten oder dergleichen gespeichert werden können, und
wobei die Speicherbereiche zwei bis vier als RAM ausgebildet sind,
wobei der zweite Speicherbereich zur Speicherung von medizinischen personenspezifischen Diagnosedaten, insbesondere von Herzerkrankungen, Stoffwechselerkrankungen, Kreislauferkrankungen, Lungenerkrankungen, Infektionen, Allergien oder dergleichen vorgesehen ist,
wobei der dritte Speicherbereich zur Speicherung von medizinischen personenspezifischen Variablen, insbesondere von Laborergebnissen, Untersuchungen, Neoplasien, Röntgenbefunden, Medikamenten, Operationen, Schwangerschaft, Implantaten oder dergleichen vorgesehen ist, und
wobei der vierte Speicherbereich zur Speicherung von medizinischen personenspezifischen Rezeptdaten vorgesehen ist,
der Speicherinhalt der Speicherbereiche mit unterschiedlichen Verschlüsselungen versehen ist,
wobei die Speicherbereiche mit unterschiedlichen Zugangsberechtigungen ausgebildet sind,
wobei eine erste Zugriffsberechtigung einem Hilfspersonal bei einem Arzt nur den Lese-Zugriff auf die Daten des als ROM ausgebildeten ersten Speicherbereichs ermöglicht,
wobei eine zweite Zugriffberechtigung einem Apotheker nur den Schreib- und Lese-Zugriff auf den als RAM ausgebildeten vierten Speicherbereich, sowie den Lesezugriff auf den als ROM ausgebildeten ersten Speicherbereich ermöglicht, und
wobei eine dritte Zugangsberechtigung einem Arzt den Schreib- und/oder Lese-Zugriff auf alle Speicherbereiche eins bis vier ermöglicht, und
wobei die Zugangsberechtigungen insbesondere als PIN und/oder als Zugangsberechtigungskarte und/oder als Code in einem externen Kartenlesegerät und/oder als biometrische Daten ausgebildet sind, und
das als Chip ausgebildete tragbare Speicherelement (10) ferner einen Transponder (21) umfaßt,
wobei mittels des Transponders ein Vorhanden- oder Nichtvorhandensein des Speicherelements (10) feststellbar ist, und
wobei mittels des Transponders die auf dem Speicherelement (10) gespeicherten Daten auslesbar sind.

2. Verfahren zum Beschreiben und/oder Lesen von medizinischen personenspezifischen Daten eines ersten als Chip ausgebildeten Speicherelements (15, 25) nach Anspruch 1, wobei zum Lesen und/oder Beschreiben von Daten aus wenigstens einem Speicherbereich (16 bis 19) des ersten Speicherelements (15, 25), die wenigstens teilweise durch wenigstens eine Zugangsberechtigung gesichert sind, eine Zugangsberechtigung einem Speicherbereich (27, 28) eines zweiten Speicherelements (15, 26) zugeführt wird und mit wenigstens einer auf dem ersten Speicherelement (15, 25) vorgebbaren Zugangsberechtigung verglichen wird, wobei bei Übereinstimmung der Zugangsberechtigung mit wenigstens einer der vorgebbaren Zugangsberechtigungen der Speicherbereich (16 bis 19) des ersten Speicherelements (15, 25), der dieser Zugangsberechtigung zugeordnet ist, freigegeben wird.

3. Verfahren nach Anspruch 2, **dadurch gekennzeichnet, daß** zum Darstellen der freigegebenen Daten die Daten aus dem ersten Speicherelement (15, 25) in einen weiteren Speicherbereich (28, 27) des zweiten Speicherelements (15, 26) gelesen werden und einer Anzeigeeinheit (30) zugeführt werden.

4. Verfahren nach Anspruch 2 und/oder 3, **dadurch gekennzeichnet, daß** zum Beschreiben des freigegebenen Speicherbereichs (16 bis 19) des ersten Speicherelements (15, 25) Daten in das zweite Speicherelement (15, 26) eingegeben werden und von dort auf das erste Speicherelement (15, 25) in den freigegebenen Speicherbereich (16 bis 19) übertragen und gespeichert werden.

5. Verwendung eines Speicherelements nach Anspruch 1 in einem System zur Erfassung und Speicherung medizinischer personenspezifischer Daten mit einem als tragbare Karte ausgebildeten Speicherelement (10) mit einem als Chip ausgebildeten Speicher (25) nach Anspruch 1, weiter umfassend einen Computer (12) und eine Verbindungseinrichtung (20), wobei das Speicherelement (10) mit der Verbindungseinrichtung (20) derart in Eingriff bringbar ist, daß Daten von dem Speicherelement (10) in den Computer (12) einlesbar sind und/oder von dem Computer (12) auf das Speicherelement schreibbar sind, wobei ferner ein Zugangselement (11) vorgesehen ist, mittels dem Daten zum Computer (12) und/oder über den Computer (12) zur Verbindungseinrichtung übermittelbar sind, wobei die Daten wenigstens einen Teil der Zugangsberechtigung aufweisen, und wobei ein weiterer Teil der Zugangsberechtigung über ein Kommunikationsmittel (13) in Form von Daten dem Computer (12) und/oder über den Computer (12) zur Verbindungseinrichtung (20) übermittelbar ist.

6. Verwendung eines Speicherelements nach Anspruch 1 in einem System nach Anspruch 5, **dadurch gekennzeichnet, daß** Mittel zur Datenfernübertragung vorgesehen sind.

7. Verwendung eines Speicherelements nach Anspruch 1 in einem System nach Anspruch 5 oder 6 **dadurch gekennzeichnet, daß** ein als Zentralcomputer ausgebildeter Computer (14) vorgesehen ist, der verschiedene Speicherbereiche (16 bis 19) auf einem Computerspeicherelement (15) beinhaltet, wobei die Speicherelemente zur Speicherung von medizinischen personenspezifischen Stammdaten, Diagnosedaten und Rezeptdaten vorgesehen sind, und wobei den verschiedenen Speicherbereichen (16 bis 19) wenigstens teilweise unterschiedliche Zugangsberechtigungen zugeordnet sind, ein tragbares, als Karte ausgebildetes Speicherelement (10) mit einem als Chip ausgebildeten Speicher (25), auf dem Daten speicherbar sind, wobei die Daten personenspezifische Stammdaten sind, und eine Verbindungseinrichtung (20), wobei das tragbare Speicherelement (10) mit der Verbindungseinrichtung (20) derart in Eingriff bringbar ist, daß Daten aus dem tragbaren Speicherelement (10) in den ersten Computer (14) übertragbar sind, wobei mittels Eingabe oder Vorliegen der entsprechenden Zugangsberechtigung die personenspezifischen Daten aus einem der jeweiligen Zugangsberechtigung entsprechenden Bereich (16 bis 19) des Computerspeicherelements (15) lesbar und/oder veränderbar sind.

8. Verwendung eines Speicherelements nach Anspruch 1 in einem System nach Anspruch 7, **dadurch gekennzeichnet, daß** mit den Mitteln zur Datenfernübertragung die Daten aus dem als Zentralcomputer ausgebildeten Computer (14) dem weiteren der Verbindungseinrichtung (20) zugeordnetem zweiten Computer (12) übermittelbar sind.

9. Verwendung eines Speicherelements nach Anspruch 1 in einem System nach einem oder mehreren der Ansprüche 5 bis 8, **dadurch gekennzeichnet, daß** mittels der Verbindungseinrichtung (20) zum Erkennen der Daten auf dem tragbaren Speicherelement (10) oder einem weiteren Element ein elektromagnetisches Signal aussendbar und empfangbar ist.

## Claims

1. Memory element (25) for storing person-specific medical data with at least two memory areas, wherein at least one memory area is a ROM and at least one memory area is a RAM, wherein different access authorisations are assigned to the memory areas, wherein the memory element (25) is in one piece, and wherein the memory element (25) is arranged on a flat structure (10) configured as a smart card,
**characterised in that**
the memory element (25) is divided into four memory areas,
• wherein the first memory area is configured as a ROM, in which person-specific master data, in particular name, address, health insurance data, data concerning next of kin, data concerning general practitioner, biometric data or the like can be stored, and
wherein memory areas two to four are configured as a RAM,
• wherein the second memory area is provided for storage of person-specific medical diagnostic data, in particular relating to heart conditions, metabolic diseases, circulatory diseases, lung diseases, infections, allergies or the like,
• wherein the third memory area is provided for the storage of person-specific medical variables, in particular laboratory results, examinations, neoplasia, X-ray results, medications, operations, pregnancy, implants or the like, and
• wherein the fourth memory area is provided for the storage of person-specific medical prescription data,
the memory content of the memory areas is provided with different encryptions,
wherein the memory areas are configured with different access authorisations,
• wherein a first access authorisation allows a doctor's auxiliary only read access to the data of the first memory area configured as a ROM,
• wherein a second access authorisation allows a pharmacist only write and read access to the fourth memory area configured as a RAM and also read access to the first memory area configured as a ROM, and
• wherein a third access authorisation allows a doctor write and/or read access to all memory areas one to four, and
wherein the access authorisations are configured in particular as PIN and/or as access authorisation card and/or as code in an external card reader and/or as biometric data, and
the portable memory element (10) configured as a chip additionally comprises a transponder (21),
• wherein a presence or absence of the memory element (10) can be established by means of the transponder, and
• wherein the data stored on the memory element (10) can be read by means of the transponder.

2. Method for writing and/or reading person-specific medical data of a first memory element (15, 25) configured as a chip according to Claim 1, wherein for reading and/or writing data from at least one memory area (16 to 19) of the first memory element (15, 25), which are at least partially secured by at least one access authorisation, an access authorisation is passed to a memory area (27, 28) of a second memory element (15, 26) and is compared to at least one predefinable access authorisation on the first memory element (15, 25), wherein when the access authorisation is consistent with at least one of the predefinable access authorisations, the memory area (16 to 19) of the first memory element (15, 25) assigned to this access authorisation is released.

3. Method according to Claim 2, **characterised in that** to display the released data, the data from the first memory element (15, 25) are read into a further memory area (28, 27) of the second memory element (15, 26) and passed to a display unit (30).

4. Method according to Claim 2 and/or 3, **characterised in that** to write the released memory area (16 to 19) of the first memory element (15, 25), data are input into the second memory element (15, 26) and from there are transferred to the first memory element (15, 25) into the released memory area (16 to 19) and stored.

5. Use of a memory element according to Claim 1 in a system for collecting and storing person-specific medical data with a memory element (10) configured as a portable card with a memory element (25) configured as a chip according to Claim 1, further comprising a computer (12) and a connection means (20), wherein the memory element (10) can be brought into engagement with the connection means (20) in such a manner that data from the memory element (10) can be read into the computer (12) and/or can be written by the computer (12) onto the memory element, wherein an access element (11) is additionally provided, by means of which data can be transferred to the computer (12) and/or via the computer (12) to the connection means, wherein the data have at least a part of the access authorisation, and wherein a further part of the access authorisation can be transferred in the form of data via a communication means (13) to the computer (12) and/or via the computer (12) to the connection means (20).

6. Use of a memory element according to Claim 1 in a system according to Claim 5, **characterised in that** means for remote data transfer are provided.

7. Use of a memory element according to Claim 1 in a system according to Claim 5 or 6, **characterised in that** a computer (14) configured as a central computer is provided, which contains different memory areas (16 to 19) on a computer memory element (15), wherein the memory elements are provided for the storage of person-specific medical master data, diagnostic data and prescription data, and wherein at least partially different access authorisations are assigned to the different memory areas (16 to 19), a portable memory element (10) configured as a card with a memory (25) configured as a chip, on which data can be stored, [is provided?], wherein the data are person-specific master data, and a connection means (20) [is provided?], wherein the portable memory element (10) can be brought into engagement with the connection means (20) in such a manner that data from the portable memory element (10) can be transferred into the first computer (14), wherein by means of input or through the presence of the corresponding access authorisation, the person-specific data from a region (16 to 19) of the computer memory element (15) corresponding to the respective access authorisation can be read and/or changed.

8. Use of a memory element according to Claim 1 in a system according to Claim 7, **characterised in that** the data can be transferred from the computer (14) configured as a central computer to the further second computer (12) assigned to the connection means (20) by the means for remote data transfer.

9. Use of a memory element according to Claim 1 in a system according to one or more of Claims 5 to 8, **characterised in that** an electromagnetic signal can be emitted and received by means of the connection means (20) for recognition of the data on the portable memory element (10) or a further element.

## Revendications

1. Elément de mémoire (25) pour le stockage de données personnelles médicales avec au moins deux zones de mémoire, dans lequel au moins une zone de mémoire est une MÉMOIRE MORTE et au moins une zone de mémoire est une MÉMOIRE VIVE, dans lequel différentes autorisations d'accès sont associées aux zones de mémoire, dans lequel l'élément de mémoire (25) est monobloc et dans lequel l'élément de mémoire (25) est disposé sur un objet plat (10) réalisé sous forme de carte à puce,
**caractérisé en ce que**
l'élément de mémoire (25) est divisé en quatre zones de mémoire,
dans lequel la première zone de mémoire est réalisée sous forme de MÉMOIRE MORTE dans laquelle des donnée personnelles de base, en particulier le nom, l'adresse, les coordonnées de la caisse d'assurance maladie, les données concernant le parent le plus proche, les données sur le médecin de famille, des données biométriques ou d'autres données de ce genre, peuvent être stockées, et
dans lequel les zones de mémoire deux à quatre sont réalisées sous forme de MÉMOIRE VIVE,
dans lequel la deuxième zone de mémoire est prévue pour le stockage de données cliniques médicales personnelles, en particulier des maladies cardiaques, des maladies métaboliques, des maladies circulatoires, des maladies pulmonaires, des infections, des allergies ou d'autres données de ce genre,
dans lequel la troisième zone de mémoire est prévue pour le stockage de variables médicales personnelles, en particulier des résultats de laboratoire, des examens, des néoplasies, des examens radiologiques, des médicaments, des opérations, des grossesses, des implants ou d'autres variables de ce genre, et
dans lequel la quatrième zone de mémoire est prévue pour le stockage de données sur les prescriptions médicales personnelles,
le contenu de la mémoire des zones de mémoire étant muni de différents codages,
dans lequel les zones de mémoire sont réalisées avec différentes autorisations d'accès,
dans lequel une première autorisation d'accès autorise uniquement l'accès en lecture aux données de la première zone de mémoire réalisée sous forme de MÉMOIRE MORTE à un personnel auxiliaire chez un médecin,
dans lequel une deuxième autorisation d'accès autorise uniquement l'accès en écriture et en lecture aux données de la quatrième zone de mémoire réalisée sous forme de MÉMOIRE VIVE ainsi que l'accès en lecture à la première zone de mémoire réalisée sous forme de MÉMOIRE MORTE à un pharmacien, et
dans lequel une troisième autorisation d'accès autorise l'accès en écriture et/ou en lecture à toutes les zones de mémoire une à quatre à un médecin, et
dans lequel les autorisations d'accès sont réalisées, en particulier, sous forme de PIN et/ou de carte d'autorisation d'accès et/ou de code dans un lecteur de carte externe et/ou de données biométriques, et
l'élément de mémoire (10) portable réalisé sous forme de puce comprend, en outre, un transpondeur (21),
dans lequel une présence ou une absence de l'élément de mémoire (10) peut être déterminée à l'aide du transpondeur, et
dans lequel les données stockées sur l'élément de mémoire (10) peuvent être lues à l'aide du transpondeur.

2. Procédé pour l'écriture et/ou la lecture de données personnelles médicales d'un premier élément de mémoire (15, 25) réalisé sous forme de puce selon la revendication 1, dans lequel, pour la description et/ou la lecture de données à partir d'au moins une zone de mémoire (16 à 19) du premier élément de mémoire (15, 25), qui sont sécurisées au moins partiellement par au moins une autorisation d'accès, une autorisation d'accès est envoyée à une zone de mémoire (27, 28) d'un deuxième élément de mémoire (15, 26) et comparée avec au moins une autorisation d'accès prédéterminable dans le premier élément de mémoire (15, 25), moyennant quoi, en cas de correspondance entre l'autorisation d'accès et au moins l'une des autorisations d'accès prédéterminables, la zone de mémoire (16 à 19) du premier élément de mémoire (15, 25) à laquelle cette autorisation d'accès est associée est débloquée.

3. Procédé selon la revendication 2, **caractérisé en ce que**, pour représenter les données débloquées, les données provenant du premier élément de mémoire (15, 25) sont lues dans une autre zone de mémoire (28, 27) du deuxième élément de mémoire (15, 26) et sont envoyées à une unité d'affichage (30).

4. Procédé selon la revendication 2 et/ou 3, **caractérisé en ce que**, pour écrire la zone de mémoire (16 à 19) débloquée du premier élément de mémoire (15, 25), les données sont entrées dans le deuxième élément de mémoire (15, 26) et, de là, elles sont transférées au premier élément de mémoire (15, 25) dans la zone de mémoire (16 à 19) débloquée et stockées.

5. Utilisation d'un élément de mémoire selon la revendication 1 dans un système pour la saisie et le stockage de données personnelles médicales avec un élément de mémoire (10) réalisé sous forme de carte portable avec une mémoire (25) réalisée sous forme de puce selon la revendication 1, comprenant, en outre, un ordinateur (12) et un dispositif de liaison (20), dans lequel l'élément de mémoire (10) peut être mis en contact avec le dispositif de liaison (20) de telle manière que les données de l'élément de mémoire (10) peuvent être lues dans l'ordinateur (12) et/ou peuvent être écrites par l'ordinateur (12) sur l'élément de mémoire, dans lequel il est prévu, en outre, un élément d'accès (11) à l'aide duquel les données peuvent être transférées à l'ordinateur (12) et/ou au dispositif de liaison par l'intermédiaire de l'ordinateur (12), dans lequel les données comportent au moins une partie de l'autorisation d'accès et dans lequel une autre partie de l'autorisation d'accès peut être transférée par un moyen de communication (13) sous forme de données à l'ordinateur (12) et/ou par l'intermédiaire de l'ordinateur (12) au dispositif de liaison (20).

6. Utilisation d'un élément de mémoire selon la revendication 1 dans un système selon la revendication 5, **caractérisé en ce qu'**il est prévu des moyens pour le transfert à distance des données.

7. Utilisation d'un élément de mémoire selon la revendication 1 dans un système selon la revendication 5, **caractérisé en ce qu'**il est prévu un ordinateur (14) réalisé sous forme d'ordinateur central qui contient différentes zones de mémoire (16 à 19) sur un élément de mémoire d'ordinateur (15), dans lequel les éléments de mémoire sont prévus pour le stockage de données de base personnelles médicales, de données cliniques et de données sur les prescriptions, et dans lequel des autorisations d'accès au moins partiellement différentes sont associées aux différentes zones de mémoire (16 à 19), un élément de mémoire portable (10) réalisé sous forme de carte avec une mémoire (25) réalisée sous forme de puce sur laquelle les données peuvent être stockées, dans lequel les données sont des données de base personnelles, et un dispositif de liaison (20), dans lequel l'élément de mémoire portable (10) peut être mis en contact avec le dispositif de liaison (20) de telle manière que les données de l'élément de mémoire portable (10) peuvent être transférées dans le premier ordinateur (14), dans lequel, à l'aide de l'entrée ou de la présence de l'autorisation d'accès correspondante, les données personnelles provenant d'une zone (16 à 19) correspondant à l'autorisation d'accès concernée de l'élément de mémoire de l'ordinateur (15) peuvent être lues et/ou modifiées.

8. Utilisation d'un élément de mémoire selon la revendication 1 dans un système selon la revendication 7, **caractérisé en ce que** les données provenant de l'ordinateur (14) réalisé sous forme d'ordinateur central peuvent être transférées au deuxième ordinateur (12) associé au dispositif de liaison (20) à l'aide des moyens pour le transfert à distance des données.

9. Utilisation d'un élément de mémoire selon la revendication 1 dans un système selon une ou plusieurs des revendications 5 à 8, **caractérisé en ce qu'**un signal électmémoire morteagnétique peut être envoyé et reçu à l'aide du dispositif de liaison (20) pour la reconnaissance des données sur l'élément de mémoire portable (10) ou sur un autre élément.
